# EUROPEAN PATENT APPLICATION

(11) **EP 0 935 133 A2**
(43) Date of publication of application: **11.08.1999**
(21) Application number: 99102149.4
(22) Date of filing: 03.02.1999
(51) Int. Cl.: G01N 21/85, G01N 1/00

(54) **Powder inspecting apparatus with electrostatic feeding of sample**

(30) Priority: 04.02.1998 JP 2315598
(71) Applicant: MINOLTA CO., LTD., Chuo-Ku, Osaka-shi, Osaka-fu (JP)
(72) Inventor: Ishida, Kiyoshi c/o Minolta Co., Ltd., Chuo-ku, Osaka-shi, Osaka-fu (JP); Takahashi, Makoto c/o Minolta Co., Ltd., Chuo-ku, Osaka-shi, Osaka-fu (JP)
(74) Representative: Glawe, Delfs, Moll & Partner

(57) **Abstract**

An inspecting apparatus is provided with a dielectric drum, a first charging device for charging the dielectric drum, a second charging device for charging powder. Charged powder is electrostatically attracted on the dielectric drum to form a thin layer of powder on the dielectric drum. An image of powder on the dielectric drum is picked up, and processed to judge presence of foreign matter in the powder.

## Description

This application is based on patent application No. 10-23155 filed in Japan, the contents of which is hereby incorporated by reference.

This invention relates to an inspecting apparatus for automatically inspecting powder.

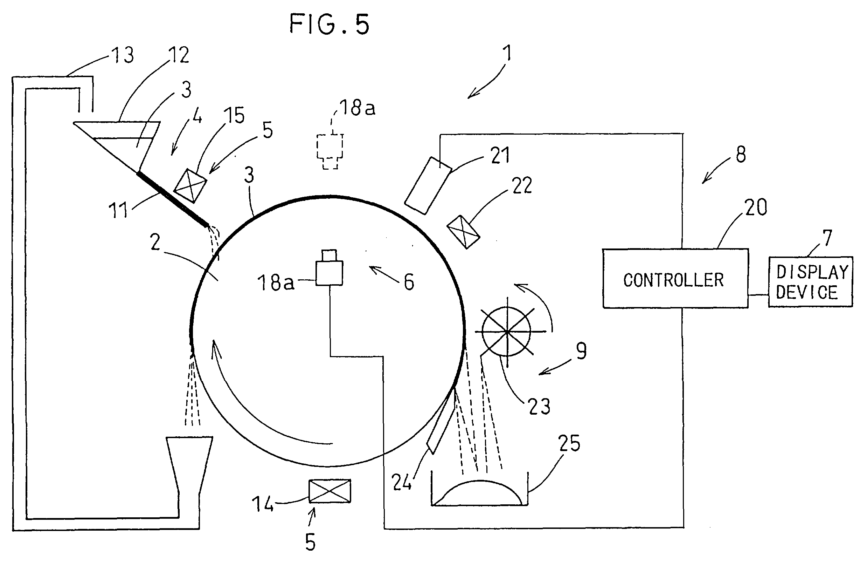

A conventional inspecting apparatus of this type which employs an automatic inspecting technique for inspecting the presence or absence of foreign matters by continuously supplying a specified amount of powder onto a conveyor while giving a vibration to the powder to form a thin layer of power is shown in FIG. 5.

In FIG. 5 showing a construction of this construction inspecting apparatus, a plurality of hoppers 52 filled with white powder 51 to be inspected are arranged side by side. Below the bottom openings of the hoppers is arranged an auxiliary hopper having an open upper end and freely movable to any of the bottom openings of the hoppers 52. A vibrating feeder 54 is provided to be continuous with the bottom opening of the auxiliary hopper 53. The white powder 51 in the hopper 52 is received onto the vibrating feeder 54 via the auxiliary hopper 53 by a specified amount by the vibration given from the vibrating feeder 54, and forms a flat layer of powder. The white powder 51 in the vibrating feeder 54 is further fed by falling onto a belt conveyor 55 arranged below a discharge end of the vibrating feeder 54. Above this discharge end is arranged a static electricity remover 56 for removing the static electricity of the powder 51 created by the vibration and the like.

A squeegee 57 is arranged downstream from a dropping point of the powder 51 on the belt conveyor 55 with respect to a direction of conveyance so as to further level the layer of the white powder 51 fallen and heaped on the belt conveyor 55. In a position above the belt conveyor 55 downstream from the squeegee 57 is arranged a reflection type gas laser scanning detector 60 comprised of a gas laser scanner 58 and a photodetector 59. A laser beam from the gas laser scanner 58 is reflected by the powder 51 to be incident on the photodetector 59. The incident light is photoelectrically converted into an electrical signal to be inputted to a controller 61.

At this time, if there is a colored foreign matter in the outer surface of the layer of the white powder 51 on the belt conveyor 55, it causes a large change in the amount of reflected light, which is detected by the controller 61 and processed as defect information.

Further, a pulse generation roller 62 is disposed on the upper surface of the downstream end of the belt conveyor 55 for inspecting a moved amount of the powder 51 by the belt conveyor 55. This moved amount is processed as moved distance information by the controller 61. The moved distance information and the defect information of the colored foreign matter are processed by a microcomputer and displayed as foreign matter information in a calculation display device 63 after a coordinate along a widthwise direction of scanning and a coordinate along the conveyance direction are added thereto. Based on the display content of the calculation display device 63, a portion of the powder where the foreign matter is present is removed by vacuum suction.

In the aforementioned conventional construction, the powder 51 is leveled by the squeegee 57 after being scattered onto the belt conveyor 55 by the specified amount by the vibration of the vibrating feeder 54. In many cases, the powder 51 on the belt conveyor 55 is not in a uniform thin layer, which presents a problem that foreign matters are buried in the layer of the powder 51 and escape the inspection. Further, the squeegee 57 as a height restricting blade having a specified height is provided at a right angle to the conveyance direction of the powder 51 and in a position above the belt conveyor 55 and at a predetermined distance therefrom. Since the squeegee 57 as a blade exerts a pressure on powder particles, there is a problem that the powder particles may be broken depending upon the type of the material of the powder 51. Further, even at a previous stage, the vibration of the vibrating feeder 54 stirs up the powder 51. This causes a problem that the stirred-up powder particles may attach to a lens for the inspection or enter a precision apparatus for the calculation, thereby damaging it.

It is an object of the present invention to provide an inspecting apparatus which has overcome the problems residing in the prior art.

According to an aspect of the invention, an inspecting apparatus for inspecting powder, comprises: an inspection base; an electrostatic attraction device which permits powder to be electrostatically attracted on the inspection base; and an optical inspecting device which executes an optical inspection to powder electrostatically attracted on the inspection base.

The electrostatic attraction device may be provided with a first charging device for charging the inspection base to have one polarity.

The electrostatic attraction device may be further provided with a second charging device which charges powder to have the opposite polarity.

The optical inspecting device may be provided with an image pickup device for picking up an image of powder on the inspection base, whereby detecting foreign matter based on a picked up image of powder.

The optical inspecting device may be provided with an optical sensor for generating optical characteristics of foreign matter and powder, whereby detecting foreign matter by comparison of optical characteristics of foreign matter with that of the powder.

The optical sensor may be a reflection type optical sensor. The optical sensor may be a transmission type optical sensor.

It may be appreciated to further provide a charge removing device for canceling the electrostatic attraction of powder on the inspection base after the inspection.

It may be appreciated to further provide a foreign matter remover for removing foreign matter from the inspection base in accordance with a result of the inspection.

The inspection base may be a transparent glass. Also, the inspection base may be made of a dielectric material.

According to another aspect of the invention, an inspecting apparatus comprises: an inspection base; a charging device which charges the inspection base to have one polarity; a supply unit which supplies powder to be inspected to the charged inspection base; and an inspection unit which executes an optical inspection for powder on the inspection base.

It may be appreciated to further provide a charging device for charging powder being supplied to the inspection base to have the opposite polarity.

It may be appreciated to further provide a charge removing device for canceling the charge of the inspection base after the optical inspection, and a collection unit for collecting inspected powder from the inspection base.

The supply unit may be provided with a recycler for collecting powder having not been electrostatically attracted to the inspection base for another supply.

The inspection unit may be provided with an image pickup unit which picks up an image of powder on the inspection base, and an image processing unit for processing the picked up image to detect foreign matter in the powder.

According to another still aspect of the invention, a method for inspecting powder, comprises the steps of: charging an inspection base to have one polarity, supplying powder to the charged inspection base, and performing an optical inspection for powder on the inspection base.

It may be appreciated to add the step of charging powder being supplied to the inspection base to have the opposite polarity.

It may be appreciated to add the step of removing the charge of the inspection base after the optical inspection.

It may be appreciated to add the step of collecting the inspected powder from the inspection base.

It may be appreciated to add the step of removing foreign matter from the inspected powder in accordance with a result of the optical inspection.

These and other objects, features and advantages of the present invention will become more apparent upon a reading of the following detailed description and accompanying drawings in which:
FIG. 1 is a diagram showing a construction of an inspecting apparatus according to one embodiment of the invention;

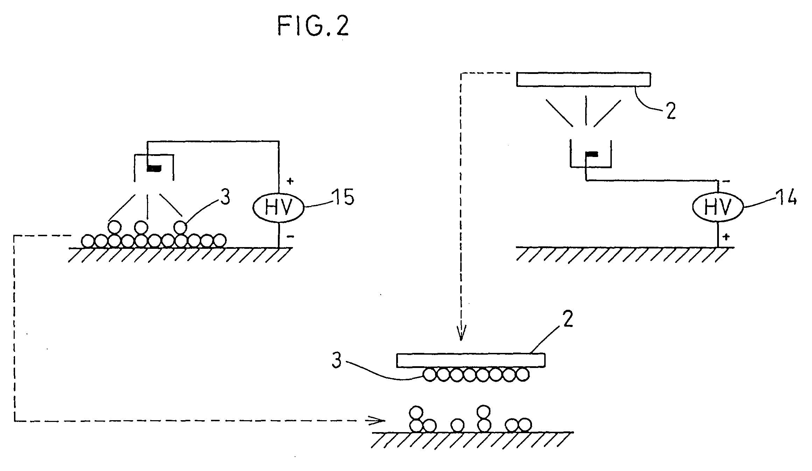

FIG. 2 is a diagram showing a mechanism for thinning a layer of powder in the inspecting apparatus of FIG. 1;
FIG. 3 is a flowchart showing an operation of a controller provided in the inspecting apparatus;
FIG. 4 is a flowchart showing an image processing operation of an image processing unit provided in the inspecting apparatus;
FIG. 5 is a diagram showing a construction of a first modified inspecting apparatus;
FIG. 6 is a diagram showing a construction of a second modified inspecting apparatus; and

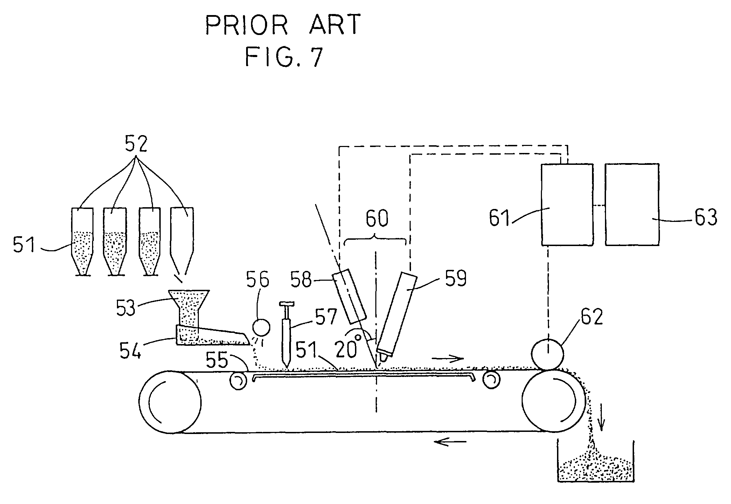

FIG. 7 is a construction diagram of a conventional inspecting apparatus.

An inspecting apparatus embodying the invention is described with reference to the accompanying drawings.

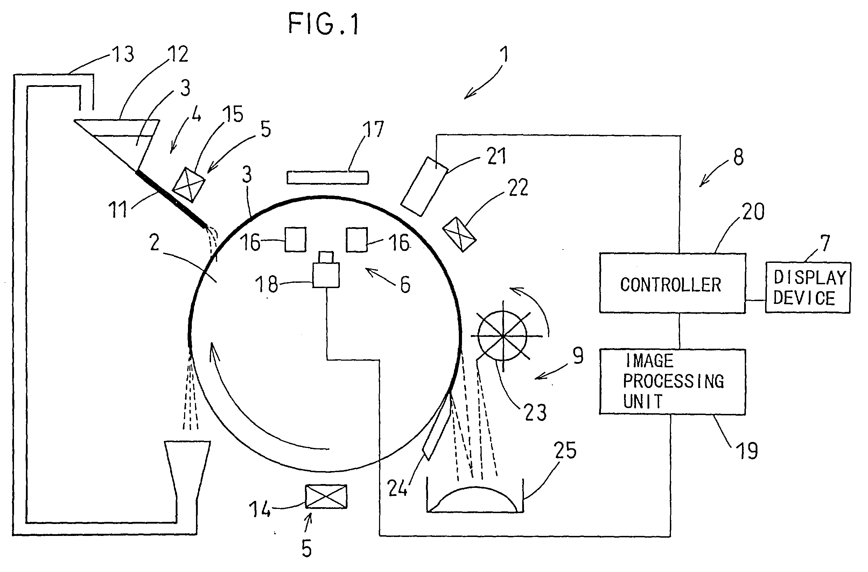

FIG. 1 shows a construction of the inspecting apparatus. In FIG. 1, the inspecting apparatus 1 includes an dielectric drum 2 as an dielectric inspection base, a powder supply device 4 for supplying powder 3 onto the dielectric drum 2, an electrostatic attraction device 5, an optical inspecting device 6, a display device 7, a foreign matter removal controlling system 8, and a powder collecting device 9. The electrostatic attraction device 5 electrostatically attracts the powder 3 supplied by the powder supply device 4 to the dielectric drum 2. The optical inspecting device 6 optically inspects a group of particles formed into a thin layer by being electrostatically attracted to the dielectric drum 2. The foreign matter removable controlling system 8 displays an inspection result of the optical inspecting device 6 in the display device 7 and, in the case that a foreign matter is detected in the powder 3 in the thin layer, executes such a control as to remove this foreign matter together with the powder 3 surrounding it. The powder collecting device 9 collects the satisfactory powder 3 after the inspection by the optical inspecting device 6 by separating it from the dielectric drum 2. In other words, the inspecting apparatus 1 forms the powder into a thin layer, detects and removes a foreign matter in the powder layer.

The dielectric drum 2 is a cylinder made of a transparent glass. The powder supply device 4 supplies the powder 3 onto the dielectric drum 2 over a specified width at a position downstream from the peak of the cylindrical dielectric drum 2 rotating in a clockwise direction.

The powder supply device 4 includes a guide member 11, a scattering device 12, and a powder recycle duct 13 as a powder recycle means. The guide member 11 supplies the powder 3 onto the dielectric drum 2 over the specified width by dropping it. The scattering device 12 drops the powder 3 onto the guide member 11 through its bottom opening. The powder recycle duct 13 collects the powder 3, which has dropped from the guide member 11 onto the dielectric drum 2, but not been electrostatically attracted to the dielectric drum 2 by receiving it at the bottom, and supplies the collected powder 3 into the scattering device 12 through its upper opening by dropping it.

The electrostatic attraction device 5 includes first and second charging devices 14, 15, taking advantage of a corona discharge. The first charging device 14 is arranged below the bottom of the dielectric drum 2 for charging the surface of the dielectric drum 2 over a specified width to have one polarity ((-) or (+)). The second charging device 15 is arranged above the guide member 11 for charging the powder 3 scattered on the guide member 11 to have a polarity opposite from that of the dielectric drum 2 ((+) or (-)). For example, by charging the surface of the dielectric drum 2 by the first charging device 14 to have a negative polarity and charging the powder 3 by the second charging device 15 to have a positive polarity, the powder 3 is electrostatically attracted in the form of a thin layer to the dielectric drum 2. The powder 3 not electrostatically attracted to the dielectric drum 2 falls along the curve surface of the dielectric drum 2 to be received by the powder recycle duct 13.

The optical inspecting device 6 includes a reflection type illuminating device 16, a transmission type illuminating device 17, an image pickup device 18 and an image processing unit 19. The illuminating device 16 illuminates the thin layer of powder 3 on the dielectric drum 2 having been electrostatically attracted thereto and having moved to an inspection stage at the top of the dielectric drum 2 together with the dielectric drum 2. The illuminating device 17 is arranged opposite from the illuminating device 16 for making the color of the surface of the drum 2 where the powder 3 is not attracted white. The image pickup device 18 is a CCD area sensor, CCD line sensor or the like for sensing an image of the powder 3 illuminated by the illuminating devices 16, 17. The image processing unit 19 is an image processor for detecting a foreign matter in the thin layer of powder 3 by processing the image of the powder 3 sensed by the image pickup device 18. The color of the surface of the drum 2 where the powder 3 is not attracted is made white by causing the light to transmit therethrough by the transmission type illuminating device 17. Accordingly, even if there are some portions in the thin layer of the powder 3 where no powder 3 is attracted, i.e., the surface of the dielectric drum 2 can be seen, such portions are not mistakenly detected as foreign matters.

The foreign matter removal controlling system 8 includes the display device 7 for displaying the inspection result of the optical inspecting device 6, a controller 20 for controllably driving a vacuum suction device 21 by calculating a suction timing of the vacuum suction device 21 based on the inspection result, and the vacuum suction device 21 for removing the detected foreign matter by suction. The controller 20 controllably drives the dielectric drum 2, the scattering device 12, the powder recycle duct 13, the first charging device 14, the second charging device 15, the image pickup device 18, the reflection type illuminating device 16, the transmission type illuminating device 17, a charge removing device 22, a cleaning brush 23 and a scraper blade 24, respectively, at specified timings.

The powder collecting device 9 includes the charge removing device 22 for canceling the electrostatic attraction of the thin layer of powder 3 to the dielectric drum 2 after the completion of the inspection, the cleaning bush 23 arranged behind the charge removing device 22 for sweeping off the powder 3 on the dielectric drum 2, the scraper blade 24 arranged behind the cleaning brush 23 for scraping off the powder 3 adhered to the dielectric drum 2 and a satisfactory powder collection box 25 for collecting the satisfactory powder 3 by receiving it.

The inspecting apparatus constructed as above operates as follows.

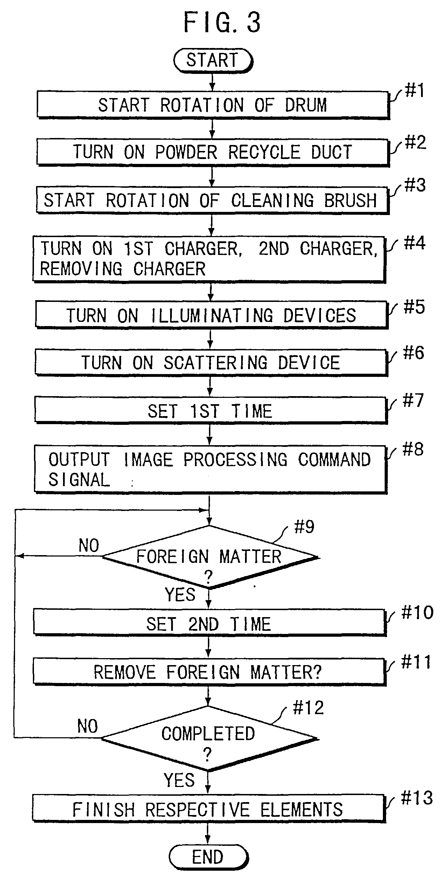

Referring to FIG. 3 showing an operation of the controller 20, the controller 20 first starts the rotation of the dielectric drum 2 (Step #1); turns the powder recycle duct 13 on (Step #2); starts the rotation of the cleaning brush 23 (Step #3), and turns the first charging device 14, the second charging device 15, the charge removing device 22 on, respectively (Step #4). Further, the controller 20 turns the reflection type illuminating device 16 and the transmission type illuminating device 17 on (Step #5); turns the scattering device 12 on (Step #6); and set a first time (Step #7).

The powder 3 as an inspection specimen stored in the scattering device 12 is scattered onto the guide member 11 through the bottom opening of the scattering device 12. While sliding down to the dielectric drum 2, the powder 3 on the guide member 11 is charged by the second charging device 15 to have a positive or negative charge. The thus charged powder 3 falls onto the slanted curved surface of the dielectric drum 2 slowly rotating clockwise.

On the other hand, the surface of the dielectric drum 2 is charged by the first charging device 14 in advance to have a polarity opposite from that of the powder 3, and the powder 3 fallen onto the slanted curved surface of the dielectric drum 2 is either electrostatically attracted in the form of a thin layer to the dielectric drum 2 or falls along the curved surface of the dielectric drum 2 without being electrostatically attracted. For example, in the case that the powder 3 is present on the thin layer of the powder 3 electrostatically attracted to the dielectric drum 2, the electrostatic attraction of the powder 3 on the layer is weak, with the result that this powder 3 slides down along the curved surface of the dielectric drum 2. In this way, out of the powder 3 fallen onto the slanted curved surface of the dielectric drum 2, the powder 3 having fallen down without being electrostatically attracted to the dielectric drum 2 is received by the powder recycle duct 13 and recycled to the scattering device 12 so as to be supplied again to the dielectric drum 2 via the guide member 11.

The thin layer of powder 3 electrostatically attracted to the dielectric drum 2 moves to the inspection stage at the top of the dielectric drum 2 as the dielectric drum 2 is rotated.

Next, when the time measures the first time set in Step #7, an image processing command signal is outputted to the image processing unit 19 (Step #8). The first time measured by the timer is initially a time necessary for the first powder layer to reach the inspection stage on the top of the dielectric drum 2 after the scattering device 12 is turned on, and is afterwards a time necessary for the dielectric drum 2 to rotate by an angle corresponding to each sensing area of the image pickup device 18.

When the controller 20 issues the image processing command signal to the image processing unit 19 in Step #8, the image processing unit 19 controls the image pickup device 18 to pick up an image of the powder 3 electrostatically attracted to the dielectric drum 2 in the form of a thin layer at the inspection stage at the top of the dielectric drum 2. The picked image of the powder 3 is transferred to and stored in a memory of the image processing unit 19, and a variety of image processings are applied to this image to determine the presence or absence of a foreign matter. In other words, the image processing unit 19 extracts an image of foreign matter by converting the picked powder image into binary data of dark and light, applies labeling, conducts an area measurement, and confirms the presence or absence of the foreign matter to be removed by comparing the measured area and a standard value.

The controller 20 waits for a foreign matter removal signal, which is outputted in the case that the image processing unit 19 determines the presence of foreign matter to be removed (Step #9). A second time is set when the foreign matter removal signal is inputted in Step #9 (Step #10). Specifically, the controller 20 judges that the foreign matter detected at the inspection stage has come to an area on the dielectric drum 2 opposite to a suction port of the vacuum suction device 21 at the time when the dielectric drum 2 is rotated only by a specified angle of rotation θ 2 by being controlled by the controller 20, and causes the vacuum suction device 21 to remove the foreign matter together with the powder 3 surrounding it by vacuum suction (Step #11).

The controller 20 further judges whether the operation of this system has been completed (Step #12) and repeatedly implements Steps #9 to #12 until the operation of this system is completed. Upon judging that the operation of this system has been completed, the controller 20 executes a processing to finish the respective elements (Step #13).

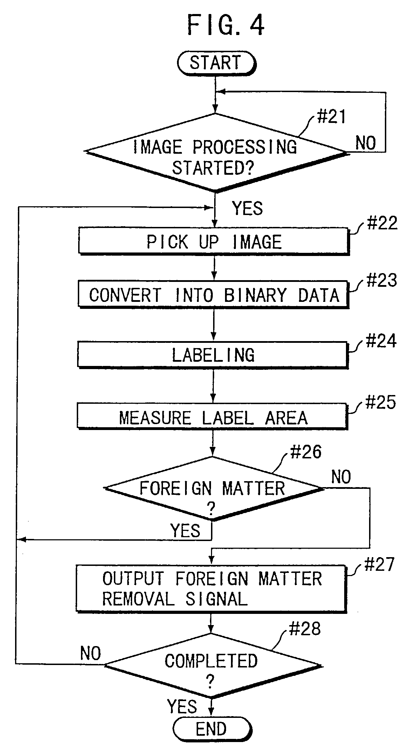

The image processing operation by the image processing unit 19 is described in detailed with reference to FIG. 4.

FIG. 4 is a flowchart showing the image processing operation by the image processing unit 19 of FIG. 1. As shown in FIG. 4, the image processing unit 19 first judges whether the image processing has been started (Step #21) and turns on a shutter of the CCD camera as the image pickup device 18 to pick up an image of the powder layer if the image processing has been started (Step #22).

Further, the image processing unit 19 extracts an image of foreign matter by converting the picked image of the powder layer into binary data of dark and light (Step #23), applies labeling (Step #24), measures the areas of the respective labels (Step #25), and determines based on comparison of the measurement values with the specified standard value whether or not there is foreign matter to be removed.

The image processing unit 19 repeats Steps #22 to #26 until judging that the foreign matter image is equal to or above the specified standard value by comparing the areas of the labels with the specified standard value. Upon judging that the foreign matter image is equal to or above the specified standard value, the image processing unit 19 outputs a foreign matter removal signal to the controller 20 (Step #27). The operations of Steps #22 to #27 are repeated until the image processing is completed (Step #28).

After the completion of the above inspection, the satisfactory powder layer having no foreign matter is applied with an electric charge removal by the charge removing device 22 to thereby lose electrostatic attraction to the dielectric drum 2. Thereafter, this powder is swept off into the satisfactory powder collection box 25 by the cleaning brush 23 to be collected as the satisfactory powder 3. Further, the powder 3 adhered to the dielectric drum 2 is scraped off into the satisfactory powder collection box 25 by the scraper blade 24 arranged behind the cleaning brush 23 to be collected as the satisfactory powder 3.

In this embodiment, there is provided the electrostatic attraction device 5 including the first charging device 14 for charging the dielectric drum 2 and the second charging device for charging the powder 3 to have a polarity opposite from that of the dielectric drum 2, so as to electrostatically attract the powder 3 in the form of a thin layer, and the optical inspecting device 6 for optically inspecting the thin layer of powder electrostatically attracted to the dielectric drum 2. Accordingly, the powder electrostatically attracted to the dielectric drum 2 forms a more uniform and stable thin layer, with the result that the optical inspection of foreign matter by the optical inspecting device 6 can be performed more highly accurately and stably.

Further, the electrostatic attraction device 5 can have a simpler construction by being formed by charging devices such as the first and second charging devices 14, 15.

Since the powder 3 electrostatically attracted to the dielectric drum 2 forms a thin layer more uniformly, the foreign matter can be detected more highly accurately and stably if an optical image processing is performed using the optical inspecting device 6.

Further, since the electrostatic attraction of the powder 3 is first canceled using the charge removing device 22 taking advantage of corona discharge and the satisfactory powder 3 is then collected using the cleaning brush 23 and scraper blade 24 as supplementary separating means, the thin layer of the satisfactory powder 3 electrostatically attracted to the dielectric drum 2 can be easily and securely collected with a simpler construction.

In this embodiment, the electrostatic attraction device 5 is comprised of the first charging device 14 for charging the dielectric drum 2 to have one polarity and the second charging device 15 for charging the powder 3 to have a polarity opposite from that of the dielectric drum 2. Alternatively, it may be comprised only of the first charging device 14 for charging the dielectric drum 2, and uncharged powder 3 may be electrostatically attracted to the dielectric drum 2 by being forcibly dielectrically polarized.

In the foregoing embodiment, the dielectric drum 2 is used as inspection base. However, it may be possible to use a drum made of an electrically conductive material instead of the dielectric drum. In this case, the conductive drum should be electrically insulated from the ground.

Further, in the foregoing embodiment, the image pickup device 18 is used to pick up an image of the powder 3 made into a thin layer by the electrostatic attraction. Instead of the image pickup device 18, there may be provided a reflection type photoelectric switch (reflection type optical sensor) or a transmission type photoelectric switch (transmission type optical sensor) to detect foreign matter by comparison the optical characteristics of foreign matter with that of the powder.

As shown in FIG. 5, specifically, a reflection type photoelectric switch 18a is provided either at a powder attracting side of the dielectric drum 2 or at an opposite side, and foreign matter in the powder 3 is detected based on a signal from this photoelectric switch.

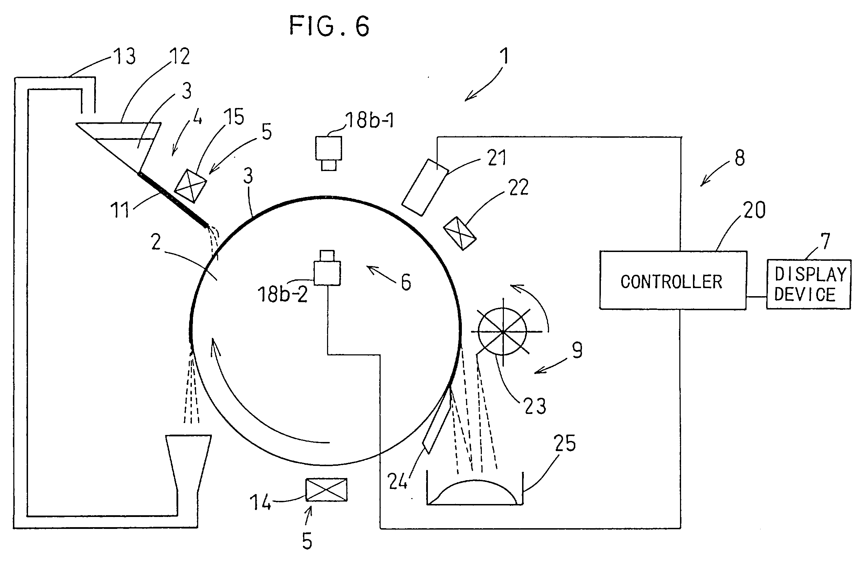

Also, as shown in FIG. 6, a light emitting element 18b-1 and a light receiving element 18b-2, which constitute a transmission type photoelectric switch, are provided so as to hold a wall of the dielectric drum 2 therebetween, and foreign matter in the powder 3 is detected based on a signal from this photoelectric switch.

In such a case, the construction including the controlling system can be simplified and manufactured at a lower cost. Further, instead of the reflection type photoelectric switch (reflection type optical sensor) or the transmission type photoelectric switch (transmission type optical sensor), a color sensor may be used.

It should be noted that the parts given with the same numerals in FIGS. 5 and 6 as FIG. 1 have identical functions to those in FIG. 1, and description of those parts is thus omitted.

Further, although the dielectric drum 2 made of a transparent glass is used as an inspection base in this embodiment, the inspection base is not limited thereto. Instead of the dielectric drum 2, an dielectric drum made of a resin or an dielectric belt for conveying the powder may be used as an inspection base. In the case of such an dielectric belt, the powder 3 is dropped onto the inclined dielectric belt and conveyed by electrostatically attracting the powder 3 to the upper surface of the dielectric belt, and the powder 3 held in the form of a uniform and thin layer by the electrostatic attraction may be optically inspected. The dielectric belt is inclined to slide the powder 3 down on the thin layer of powder 3 to obtain a thinner layer of the powder 3.

As described above, inspection is made to powder electrostatically attracted to an inspection base. Accordingly, the powder electrostatically attracted to the inspect ion base can form a more uniform and thin layer without being crushed by a pressure applied to thin the powder layer. Thus, unlike the conventional apparatus, there is no likelihood that foreign matter is buried in the powder layer to escape the inspection and the electrostatic attraction restrain ins the powder from being stirred up, thereby preventing troubles caused by the stirred-up powder (attachment to lenses and entrance into accurate equipment). Therefore, an optical inspection can be performed more highly accurately and stably.

The provision of a charging device to perform the electrostatic attraction renders the construction simpler.

Further, since powder electrostatically attracted to the inspection base forms a more uniform thin layer, the inspection can be performed more highly accurately and stably if the image processing unit is used. Such an ability to form a uniform thin layer of the powder by the electrostatic attraction enables the use of a reflection type photoelectric switch or transmission type photoelectric switch instead of the image processing unit, and the construction including the controlling system can be made simpler and can be manufactured at a lower cost.

Furthermore, there are provided a charge removing device to cancel the electrostatic attraction of the powder to the inspection base after the completion of the inspection and a separator to separate the powder from the inspection base after charges are removed. Accordingly, the thin layer of the satisfactory powder electrostatically attracted to the inspection base can be easily and securely collected with a simple construction.

The inspection base is preferably made of dielectric material. The dielectric material enhances the electrostatic attraction, and ensures a simpler construction.

Although the present invention has been fully described by way of example with reference to the accompanying drawings, it is to be understood that various changes and modifications will be apparent to those skilled in the art. Therefore, unless otherwise such changes and modifications depart from the scope of the present invention, they should be construed as being included therein.

## Claims

1. An inspecting apparatus for inspecting powder, comprising:
an inspection base (2);
an electrostatic attraction device (5) which permits powder (3) to be electrostatically attracted on the inspection base (2); and
an optical inspecting device (6) which executes an optical inspection to powder (3) electrostatically attracted on the inspection base (2).

2. An inspecting apparatus according to claim 1, wherein the electrostatic attraction device (5) includes a first charging device (14) which charges the inspection base (2) to have one polarity.

3. An inspecting apparatus according to claim 2, wherein the electrostatic attraction device (5) further includes a second charging device (15) which charges powder (3) to have the opposite polarity.

4. An inspecting apparatus according to one of claims 1 to 3, wherein the optical inspecting device (6) includes an image pickup device (18) which picks up an image of powder on the inspection base (2), whereby detecting foreign matter based on a picked up image of powder (3).

5. An inspecting apparatus according to one of claims 1 to 3, wherein the optical inspecting device (6) includes an optical sensor (18a, 18b) which generates optical characteristics of foreign matter and powder (3), whereby detecting foreign matter by comparison of optical characteristics of foreign matter with that of the powder (3).

6. An inspecting apparatus according to claim 5, wherein the optical sensor is a reflection type optical sensor (18a).

7. An inspecting apparatus according to claim 5, wherein the optical sensor is a transmission type optical sensor (18b).

8. An inspecting apparatus according to one of claims 1 to 7, further comprising a charge removing device (22) which cancels the electrostatic attraction of powder (3) on the inspection base (2) after the inspection.

9. An inspecting apparatus according to one of claims 1 to 8, wherein the inspection base (2) includes a transparent glass.

10. An inspecting apparatus according to one of claims 1 to 9, further comprising a foreign matter remover (8) which removes foreign matter from the inspection base (2) in accordance with a result of the inspection.

11. An inspecting apparatus according to one of claims 1 to 10, wherein the inspection base (2) is made of a dielectric material.
